# EUROPEAN PATENT SPECIFICATION

(11) **EP 4 087 483 B1**
(45) Date of publication and mention of the grant of the patent: **25.09.2024**
(21) Application number: 21702552.7
(22) Date of filing: 06.01.2021
(51) Int. Cl.: A61B 5/282, A61B 5/259, A61B 5/00

(54) **BIOLECTRIC SENSOR DEVICE AND METHODS**
BIOELEKTRISCHE SENSORVORRICHTUNG UND VERFAHREN
DISPOSITIF ET PROCÉDÉS DE CAPTEUR BIOÉLECTRIQUE

(30) Priority: 06.01.2020 US 202062957449 P; 31.07.2020 US 202016944230
(43) Date of publication of application: 16.11.2022
(73) Proprietor: Medtronic, Inc., Minneapolis, Minnesota 55432 (US)
(72) Inventor: GELFMAN, Daniel M., Minneapolis, Minnesota 55432 (US); NARR, Paul M., Minneapolis, Minnesota 55432 (US); SCHOTZKO, Elizabeth A., Minneapolis, Minnesota 55432 (US); DAHLBERG, Lindsey T., Minneapolis, Minnesota 55432 (US); REICH, Jordyn, Minneapolis, Minnesota 55432 (US); PARKS, Nicole, Minneapolis, Minnesota 55432 (US); PISTELLA, Maggie J., Minneapolis, Minnesota 55432 (US)
(74) Representative: Zimmermann & Partner Patentanwälte mbB
(86) International application number: PCT/US2021/012260
(87) International publication number: WO 2021/141951

(56) References cited:
- US-A1- 2012 226 131
- US-A1- 2013 248 226
- US-A1- 2015 157 225
- US-A1- 2018 271 394

## Description

### TECHNICAL FIELD

This disclosure generally relates to bioelectric sensor devices, systems, and methods that utilize such devices for sensing bioelectric data from a human body.

### BACKGROUND

Various systems and devices can be used for sensing bioelectric data from a human body. For example, multi-electrode electrocardiogram (ECG) systems can be utilized for body-surface potential mapping by recording simultaneous ECG measurements from multiple sensors or electrodes applied to selected locations of a patient's body These sensors may be included in apparatus such as vests, bands, belts, straps, patches, wearable garments, t-shirts, bras, hats (e.g., for neural signals), etc.

Some ECG systems include multiple sensors generally arranged as part of a vest that is attached to a patient. These vests can be applied to a patient's torso for receiving bioelectric signals and, in some configurations, delivering stimulating signals to the patient. Bioelectric signals from the patient are detected by the sensors and transmitted via conductive paths to a medical monitoring system or apparatus such as an ECG base unit.

For example, one type of electrode vest is described in U.S. Patent No. 6,055,448 to Anderson et al., entitled SENSOR DEVICE. The described device includes a plurality of finger-like substrate portions of a flexible dielectric material that are releasably attachable to the thoracic region of a human body.

Further, for example, U.S. Patent Publication No. 2013/0018251 to Caprio et al., entitled SENSOR DEVICE WITH FLEXIBLE JOINTS, describes a sensor device that includes a flexible dielectric substrate, a plurality of sensors distributed on the substrate, and an electrically conductive network distributed on the substrate connecting the sensors to a terminal portion of the substrate. Integrated flexible joints permit a certain amount of elasticity in and allow relative movement between at least two of the sensors when the sensor device is placed onto the human body.

Such vests are generally provided in multiple sizes to accommodate various body types and sizes of patients. For example, U.S. Patent Publication No. 2011/0190615 to Phillips et al., entitled ELECTRODE PATCH MONITORING DEVICE, describes an electrode patch monitoring device that includes an array of electrodes formed on a flexible substrate. The electrode patch monitoring device may be available in a plurality of sizes. US 2015/0 157 225 A1 is related to a bioelectric sensor device and methods.

### SUMMARY

The present invention relates to a bioelectric sensor device as defined in claim 1. Preferred embodiments of the invention are defined in the dependent claims.

The following aspects of the disclosure are useful for understanding the invention.

The techniques of this disclosure generally relate to bioelectric sensor devices, systems, and methods that include such devices. In one or more embodiments, the devices can be used for sensing bioelectric data from a human body. Further, in one or more embodiments, the devices can be used to help determine whether a patient will benefit from cardiac resynchronization therapy (CRT). If a patient is a viable candidate for such therapy, then one or more embodiments of the disclosed devices can be used to aid in placement of one or more leads of an implantable medical device and monitor cardiac activity to fine tune pacing and sensing parameters of the implanted device.

In one example, aspects of this disclosure relate to a bioelectric sensor device that includes a central portion and an arm that extends from the central portion, where at least a portion of the arm extends along an arm axis The central portion includes an anatomical alignment mark adapted to align the central portion with an anatomical feature of a lateral surface of a torso of a patient. Further, the arm is adapted to be disposed on an anterior or posterior surface of the torso. The bioelectric sensor device also includes a sensor disposed on the arm along the arm axis, and a connector electrically connected to the sensor.

In another example, aspects of this disclosure relate to a method that includes locating an anatomical feature on a lateral surface of a torso of a patient, disposing a central portion of a bioelectric sensor device on the lateral surface of the torso such that an anatomical alignment mark disposed on the central portion is aligned with the anatomical feature, and manipulating an arm of the bioelectric sensor device from an undeployed configuration to a deployed configuration, where the arm is connected to the central portion of the device. The method further includes disposing the arm on either an anterior or posterior surface of the torso of the patient, and electrically connecting a sensor disposed on the arm with a monitoring system.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a schematic side view of one embodiment of a patient monitoring system that includes a bioelectric sensor device.
FIG. 2 is a schematic plan view of one embodiment of a bioelectric sensor device.
FIG. 3 is a schematic plan view of a portion of the bioelectric sensor device of FIG. 2 disposed on lateral and posterior surfaces of a torso of a patient.
FIG. 4 is a schematic plan view of a portion of the bioelectric sensor device of FIG. 2 disposed on lateral and anterior surfaces of the torso of the patient such that the device is in a deployed configuration.
FIG. 5 is a schematic plan view the bioelectric sensor device of FIG. 2 in an undeployed configuration.
FIG. 6 is a schematic cross-section view of a portion of the bioelectric sensor device of FIG. 2.
FIG. 7 is a schematic plan view of another embodiment of a bioelectric sensor device.
FIG. 8 is a schematic plan view of another embodiment of a bioelectric sensor device.
FIG. 9 is a schematic plan view of another embodiment of a bioelectric sensor device.
FIG. 10 is a flowchart of one embodiment of a method of disposing the bioelectric sensor device of FIG. 2 on the torso of the patient.

### DETAILED DESCRIPTION

The techniques of this disclosure generally relate to bioelectric sensor devices, systems, and methods that include such devices. In one or more embodiments, the devices can be used for sensing bioelectric data from a human body. Further, in one or more embodiments, the devices can be used to help determine whether a patient will benefit from cardiac resynchronization therapy (CRT). If a patient is a viable candidate for such therapy, then one or more embodiments of the disclosed devices can be used to aid in placement of one or more leads of an implantable medical device and monitor cardiac activity to adjust pacing and sensing parameters of the implanted device.

Currently, bioelectric sensor devices such as vests and belts can be challenging for a clinician to apply to a patient as such devices tend to include somewhat rigid substrates that do not easily conform to the patient's torso. Such devices can be difficult to properly locate on the torso before application as each patient can have a unique anatomy; therefore, greater variability in the design of these devices may be desirable. Further, additional bioelectric sensor devices may need to be kept in inventor to accommodate varying shapes and sizes of patients. And improved cardiac monitoring systems can require additional electrodes disposed in unique locations that current bioelectric sensor devices cannot accommodate.

One or more embodiments of bioelectric sensor devices described herein can provide one or more advantages over current sensor devices. For example, one or more embodiments of devices can include any suitable number of electrodes that are disposed on a flexible substrate that can provide biopotential values for a variety of patient anatomies. Further, one or more embodiments of devices can include a unique design of one or more arms that can increase accuracy of placement of electrodes disposed on the one or more arms while improving patient comfort and motion management of the device as a patient moves while measurements are being taken. The device can, in one or more embodiments, include two or more portions or segments that can be connected after the two or more portions are disposed on the torso of the patient. Further, the device can be rolled-up or folded together in an undeployed configuration and disposed within a smaller package for shipping and storage and then deployed for use.

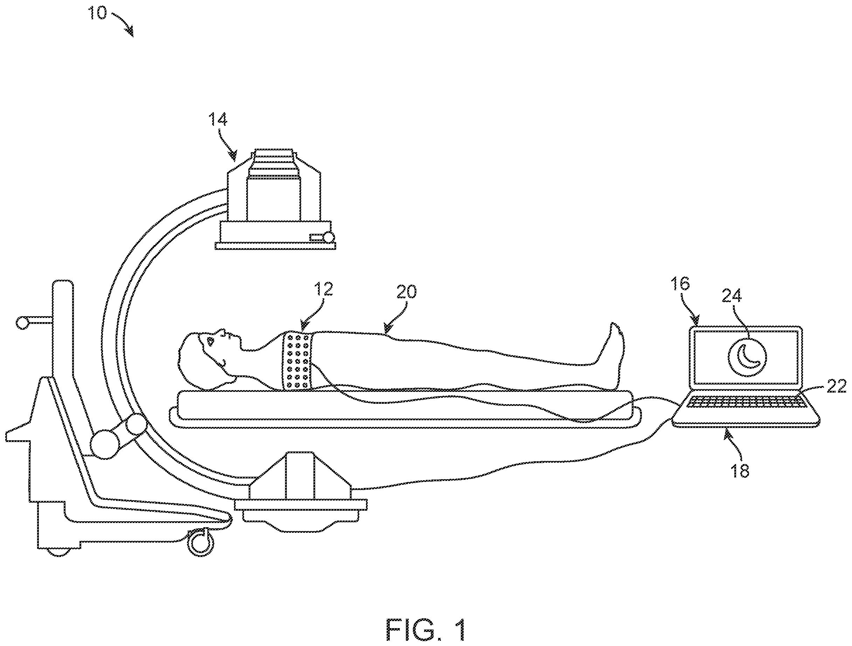

The various embodiments of bioelectric sensor devices described herein can be utilized with any suitable patient monitoring system. For example, FIG. 1 is a schematic side view of one embodiment of a patient management system 10. The system 10 includes a bioelectric sensor device 12, an imaging apparatus 14, a display apparatus 16, and a computing apparatus 18.

The sensor device 12, which can include any suitable sensor device described herein, is operatively coupled to the computing apparatus 18 to provide electrical signals from each of one or more sensors of the sensor device to the computing apparatus 18 for analysis.

The imaging apparatus 14 can be any type of imaging apparatus adapted to image, or provide images of, at least a portion of the patient in a non-invasive manner. For example, the imaging apparatus 14 may not use any components or parts that may be located within the patient to provide images of at least a portion of the patient except non-invasive tools such as contrast solution. It is to be understood that the exemplary systems, methods, and interfaces described herein may noninvasively assist a user (e.g., a physician or clinician) in selecting a location proximate a patient's heart for an implantable electrode, and after the exemplary systems, methods, and interfaces have provided noninvasive assistance, the exemplary systems, methods, and interfaces can then provide assistance to implant, or navigate, an implantable electrode or other device into the patient, e.g., proximate the patient's heart.

For example, after the exemplary systems, methods, and interfaces have provided noninvasive assistance, the exemplary systems, methods, and interfaces may then provide image guided navigation that may be used to navigate leads including electrodes, leadless electrodes, wireless electrodes, catheters, etc., within the patient's body. Further, although the exemplary systems, methods, and interfaces are described herein with reference to a patient's heart, it is to be understood that the exemplary systems, methods, and interfaces may be applicable to any other portion of the patient's body.

The imaging apparatus 14 may be configured to capture, or take, x-ray images (e.g., two-dimensional x-ray images, three-dimensional x-ray images, etc.) of a patient 20. The imaging apparatus 14 can be operatively coupled (e.g., through one or wired electrical connections, wirelessly, etc.) to the computing apparatus 18 such that the images captured by the imaging apparatus 14 may be transmitted to the computing apparatus 18. Further, the computing apparatus 18 can be adapted to control the imaging apparatus 14 to, e.g., adapt the imaging apparatus 14 to capture images, change one or more settings of the imaging apparatus 14, etc.

It will be recognized that while the imaging apparatus 14 as shown in FIG. 1 can be adapted to capture x-ray images, any other alternative imaging modality can also be used by the exemplary systems, methods, and interfaces described herein. For example, the imaging apparatus 14 may be adapted to capture images, or image data, using isocentric fluoroscopy, bi-plane fluoroscopy, ultrasound, computed tomography (CT), multi-slice computed tomography (MSCT), magnetic resonance imaging (MRI), high frequency ultrasound (HIFU), optical coherence tomography (OCT), intra-vascular ultrasound (IVUS), two dimensional (2D) ultrasound, three dimensional (3D) ultrasound, four dimensional (4D) ultrasound, intraoperative CT, intraoperative MRI, etc. Further, it is to be understood that the imaging apparatus 14 can be adapted to capture a plurality of consecutive images (e.g., continuously) to provide video frame data. In other words, a plurality of images taken over time using the imaging apparatus 14 may provide motion picture data. Additionally, the images may also be obtained and displayed in two, three, or four dimensions. In more advanced forms, four-dimensional surface rendering of the heart or other regions of the body can also be achieved by incorporating heart data or other soft tissue data from an atlas map or from pre-operative image data captured by MRI, CT, or echocardiography modalities. Image datasets from hybrid modalities, such as positron emission tomography (PET) combined with CT, or single photon emission computer tomography (SPECT) combined with CT, could also provide functional image data superimposed onto anatomical data to be used to confidently reach target locations within the heart or other areas of interest.

The display apparatus 16 and the computing apparatus 18 can be adapted to display and analyze data such as, e.g., surrogate electrical activation data, image data, mechanical motion data, etc. gathered, or collected, using the sensor device 12 and the imaging apparatus 14 to noninvasively assist a user in location selection of an implantable electrode. In one or more embodiments, the computing apparatus 18 can be a server, a personal computer, a tablet computer, a mobile device such as a smartphone, or an application run by any of these devices. The computing apparatus 18 can be adapted to receive input from input apparatus 22 and transmit output to the display apparatus 16. Further, the computing apparatus 18 may include data storage that can allow for access to processing programs or routines and/or and one or more other types of data, e.g., for driving a graphical user interface configured to noninvasively assist a user in location selection of an implantable electrode, etc.

The computing apparatus 18 can be operatively connected to the input apparatus 22 and the display apparatus 16 to, e.g., transmit data to and from each of the input apparatus 22 and the display apparatus 16. For example, the computing apparatus 18 can be electrically coupled to each of the input apparatus 22 and the display apparatus 16 using, e.g., analog electrical connections, digital electrical connections, wireless connections, bus-based connections, network-based connections, internet-based connections, etc. As described further herein, a user can provide input to the input apparatus 22 to manipulate, or modify, one or more graphical depictions displayed on the display apparatus 16 to view and/or select one or more target or candidate locations of a portion of a patient's heart as further described herein.

Although as depicted the input apparatus 22 is a keyboard, it is to be understood that the input apparatus can include any apparatus capable of providing input to the computing apparatus 18 to perform the functionality, methods, and/or logic described herein. For example, the input apparatus 22 can include a mouse, a trackball, a touchscreen (e.g., capacitive touchscreen, a resistive touchscreen, a multi-touch touchscreen, a voice-activated screen, etc.), etc. Likewise, the display apparatus 16 can include any apparatus capable of displaying information to a user, such as a graphical user interface 24 including graphical depictions of anatomy of a patient's heart, images of a patient's heart, graphical depictions of locations of one or more electrodes, graphical depictions of one or more target or candidate locations, alphanumeric representations of one or more values, graphical depictions or actual images of implanted electrodes and/or leads, etc. For example, the display apparatus 16 can include a liquid crystal display, an organic light-emitting diode screen, a touchscreen, a cathode ray tube display, etc.

The graphical user interfaces 24 displayed by the display apparatus 16 can include, or display, one or more regions used to display graphical depictions, to display images, to allow selection of one or more regions or areas of such graphical depictions and images, etc. As used herein, a "region" of a graphical user interface 24 can be defined as a portion of the graphical user interface 24 within which information may be displayed or functionality may be performed. Regions may exist within other regions, which can be displayed separately or simultaneously. For example, smaller regions may be located within larger regions, regions may be located side-by-side, etc. Additionally, as used herein, an "area" of a graphical user interface 24 can be defined as a portion of the graphical user interface 24 located with a region that is smaller than the region it is located within.

The processing programs or routines stored and/or executed by the computing apparatus 18 can include programs or routines for computational mathematics, matrix mathematics, decomposition algorithms, compression algorithms (e.g., data compression algorithms), calibration algorithms, image construction algorithms, signal processing algorithms (e.g., Fourier transforms, fast Fourier transforms, etc.), standardization algorithms, comparison algorithms, vector mathematics, or any other processing required to implement one or more exemplary methods and/or processes described herein. Data stored and/or used by the computing apparatus 18 can include, for example, image data from the imaging apparatus 14, electrical signal data from the sensor device 12, graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein, or any other data that may be necessary for carrying out the one and/or more processes or methods described herein.

In one or more embodiments, the exemplary systems, methods, and interfaces can be implemented using one or more computer programs executed on programmable computers, such as computers that include, for example, processing capabilities, data storage (e.g., volatile or non-volatile memory and/or storage elements), input devices, and output devices. Program code and/or logic described herein can be applied to input data to perform functionality described herein and generate desired output information. The output information can be applied as input to one or more other devices and/or methods as described herein or as would be applied in a known fashion.

The one or more programs used to implement the systems, methods, and/or interfaces described herein can be provided using any programmable language, e.g., a high-level procedural and/or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, at least in one embodiment, the exemplary systems, methods, and/or interfaces may be implemented using a computer readable storage medium, configured with a computer program, where the storage medium so configured causes the computer to operate in a specific and predefined manner to perform functions described herein. Further, in at least one embodiment, the exemplary systems, methods, and/or interfaces may be described as being implemented by logic (e.g., object code) encoded in one or more non-transitory media that includes code for execution and, when executed by a processor, is operable to perform operations such as the methods, processes, and/or functionality described herein.

The computing apparatus 18 can be, for example, any fixed or mobile computer system (e.g., a controller, a microcontroller, a personal computer, minicomputer, tablet computer, mobile device such as a smartphone, an application installed on any of these devices, etc.). The exact configuration of the computing apparatus 18 is not limiting, and essentially any device capable of providing suitable computing capabilities and control capabilities (e.g., graphics processing, etc.) may be used. As described herein, a digital file may be any medium (e.g., volatile or non-volatile memory, a CD-ROM, a punch card, magnetic recordable tape, etc.) containing digital bits (e.g., encoded in binary, trinary, etc.) that may be readable and/or writeable by computing apparatus 18 described herein. Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user.

In view of the above, it will be readily apparent that the functionality as described in one or more embodiments according to the present disclosure may be implemented in any manner as would be known to one skilled in the art. As such, the computer language, the computer system, or any other software/hardware which is to be used to implement the processes described herein shall not be limiting on the scope of the systems, processes or programs (e.g., the functionality provided by such systems, processes or programs) described herein.

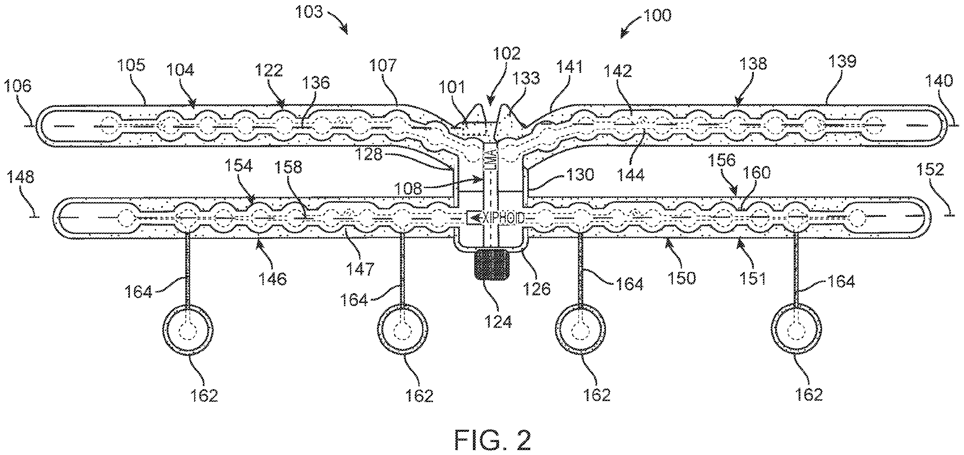

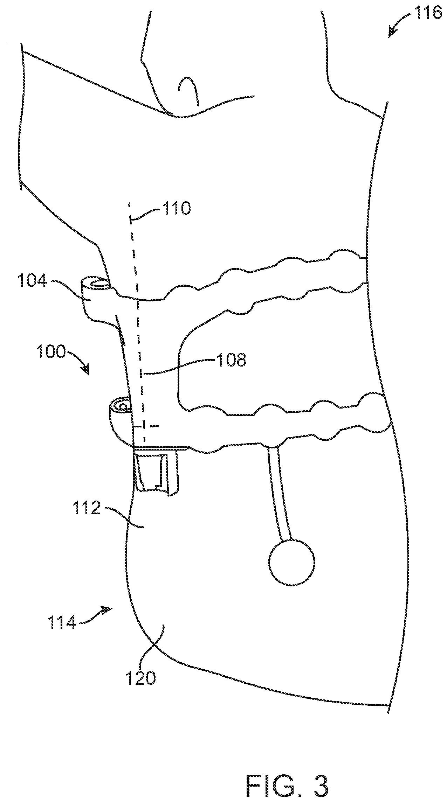

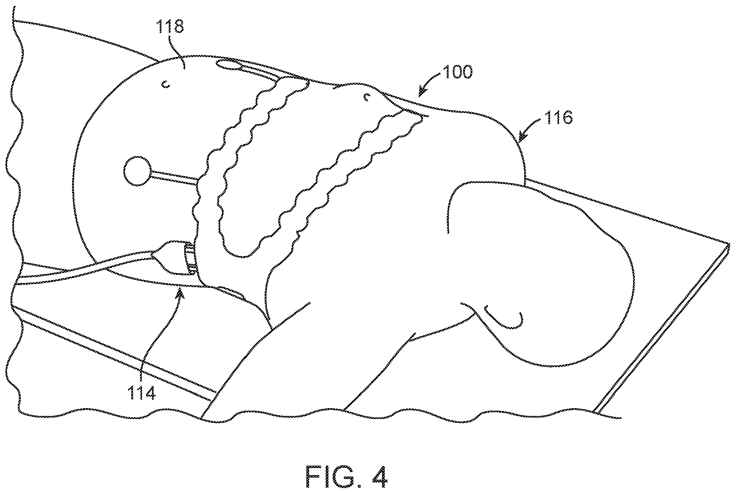

As mentioned herein, the patient monitoring system 10 can include any suitable bioelectric sensor device 12. For example, FIGS. 2-6 are various views of one embodiment of a bioelectric sensor device 100. The device 100 includes a central portion 102 and an arm 104 extending from the central portion. At least a portion 105 of the arm 104 extends along an arm axis 106. The central portion 102 includes an anatomical alignment mark 108 adapted to align the central portion with an anatomical feature 110 (FIG. 3) of a lateral surface 112 of a torso 114 of a patient 116. The arm 104 is adapted to be disposed on an anterior surface 118 (FIG. 4) or posterior surface 120 (FIG. 3) of the torso 114. The device 12 further includes one or more sensors 122 disposed on the arm 104 along the arm axis 106, and a connector 124 electrically connected to the one or more sensors.

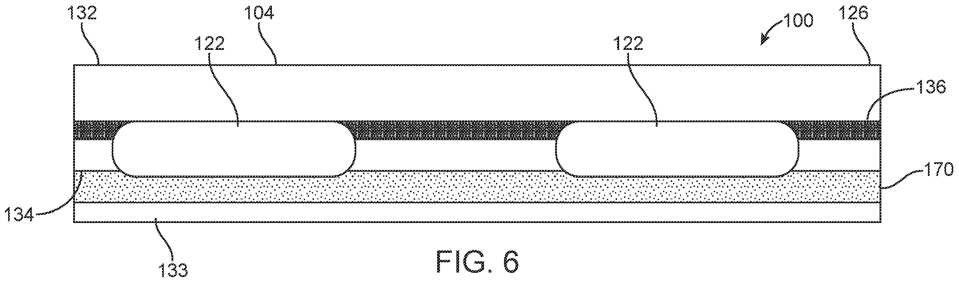

In general, the device 100 can have any suitable construction. As shown in FIG. 6, which is a schematic cross-section view of a portion of the arm 122, the device 100 can include a substrate 126 that forms at least one of the central portion 102 or the arm 104. The substrate 126 can be constructed of any suitable material or materials, e.g., polymeric, rubber, natural fiber, etc. In one or more embodiments, the substrate 126 can include at least one of polyester (e.g., MYLAR), polyethylene foam, polyester non-woven materials, cellulose rayon non-woven materials, polyethylene vinyl acetate films, polyethylene terephthalate films, thermoplastic polyurethane films, polyimide films, Spandex^{™}, or jacketed ribbon cable. In one or more embodiments, the substrate 126 can include a flexible dielectric substrate. The substrate 126 can be a single layer or multiple layers of materials. Further, the substrate 126 can be transparent, translucent, and/or opaque in one or more areas. In the embodiment illustrated in FIG. 6, the substrate includes a first major surface 132 and a second major surface 134.

The device 100 can, in one or more embodiments, include an adhesive layer 170 disposed on the second major surface 134 of the substrate 126 that faces the torso 114 of the patient 116 or on one or more electrodes 122 (FIG. 6) such that the device can be attached to skin of the patient after the device is positioned in the desired location relative to the patient. The adhesive layer 170 can include any suitable adhesive, e.g., conductive hydrogels (e.g., polyethylene glycol, etc.), carbon impregnated pressure sensitive adhesive, etc. For example, in one or more embodiments, conductive hydrogels may include cationic acrylates, including, e.g., acrylic esters of quaternary chlorides and/or sulfates or acrylic amides of quaternary chlorides. In one or embodiments, the adhesive layer 170 can include a conductive adhesive such that the one or more sensors 122 are in electrical contact with the patient 116 through the conductive adhesive. Any suitable conductive adhesive can be used for the adhesive layer,

In one or more embodiments, the adhesive layer 170 can include conductive gel disposed on the second major surface 134 of the substrate 126 and aligned with one or more sensors 122 to provide electrical contact between the sensors and skin of the patient 116, and a conductive or non-conductive adhesive disposed on the second major surface to attach the device 100 to skin of the patient. The conductive or non-conductive adhesive can be disposed in any suitable pattern on the major surface of the substrate 126, e.g., surrounding areas of conductive gel. In one or more embodiments, the adhesive layer 170 is a continuous layer disposed on the second major surface 134 of the substrate 126. In one or more embodiments, the adhesive layer 170 is discontinuous, e.g., portions or segments of the adhesive layer are aligned with the electrodes 122 and not disposed on the remainder of the second major surface 134 of the substrate 126.

Further, the device 100 can, in one or more embodiments, include a liner 133 disposed on the adhesive layer 170 such that the adhesive layer is disposed between the substrate 126 and the liner. The liner 133 can be removed before the device 100 is attached to skin of the patient 116. Any suitable liner 133 can be utilized, e.g., polymer (e.g., polyethylene, polypropylene), plastic, rubber, natural fiber, polyester, etc. In one or more embodiments, the liner 133 can include a paper backing with a coating on one or both sides of the paper, where the coating can include silicone release agents that can provide a differential release. Further, one or more embodiments of liner 133 can include indicium, images, etc., that can be utilized by a user for placement of the device 100 on the torso 114.

Returning to FIGS. 2-6, the central portion 102 of the device 100 can be a portion of the substrate 126 or formed from a different material (e.g., the same materials described herein for the substrate) and connected to the substrate using any suitable technique or techniques. Further, the central portion 102 can include any suitable number of layers. The central portion 102 of the device 100 can take any suitable shape or shapes and have any suitable dimensions. The central portion 102 can include a left edge 128 and a right edge 130. The arm 104 can extend from the left edge 128 of the central portion 102.

The central portion 102 also includes the anatomical alignment mark 108. The mark 108 can be disposed on or within the central portion 102 using any suitable technique or techniques, e.g., adhering, printing, embossing, ablating, etc. In one or more embodiments, the mark 108 can be disposed on a major surface of the central portion 102. Further, in one or more embodiments, the mark 108 can be disposed between two layers of the central portion 102.

The anatomical alignment mark 108 can include any suitable indicium, shape, pattern, etc. that is adapted to align the central portion 102 with the anatomical feature 110 of the patient 116. As shown in FIG. 2, the mark 108 includes a dashed line that is adapted to align the central portion of the device 100 with the anatomical feature 110. In one or more embodiments, the mark 108 can include a second indicium, shape, pattern, etc. that is adapted to align the central portion 102 with a second anatomical feature. In one or more embodiments, the anatomical alignment mark 108 can be adapted to align the central portion 102 of the device 100 with any suitable number of anatomical features.

Further, the central portion 102 can be aligned with any suitable anatomical feature 110 of the patient 116. In one or more embodiments, the mark 108 can be adapted to align the central portion 102 with at least one of the xiphoid, a shoulder blade, or sternum of the torso 120.

Any suitable technique or techniques can be utilized to align the central portion 102 with the anatomical feature 110. In one or more embodiments, the anatomical feature 110 can be marked with a surgical marker, and the anatomical alignment mark 108 can be disposed on the anatomical feature such that the central portion 102 is aligned with the anatomical feature. The central portion 102 can be connected to the torso 114 using any suitable technique or techniques as is further described herein.

Extending from the central portion 102 is the arm 104. The arm 104 can take any suitable shape or shapes and have any suitable dimension. In one or more embodiments, the arm 104 takes a substantially rectilinear shape along the arm axis 106. In one or more embodiments, the arm can take a serpentine shape that oscillates about the arm axis 106 as is further described herein. In one or more embodiments, the arm 104 can be formed such that it includes one or more accordion folds to accommodate various patient anatomies.

The arm 104 is adapted to be disposed on at least one of the anterior 118 or posterior 120 surface of the torso 114. Further, at least a portion 105 of the arm extends along the arm axis 106. In one or more embodiments, the entire arm 104 extends along the arm axis 106. Further, in one or more embodiments, a second portion 107 of the arm 104 does not extend along the arm axis 106.

The arm 104 can be integral with the central portion 102, i.e., the arm and the central portion are manufactured as one part. For example, the arm 104 can be formed from the substrate 126 that forms the central portion 102. In one or more embodiments, the arm 104 can be manufactured separately and connected to the central portion 102 using any suitable technique or techniques. In one or more embodiments, the arm is connected to the central portion by a fastener 101. The fastener 101 can include any suitable fastener, e.g., snaps, adhesives, hook-and-loop fasteners, clips, etc. The arm 104 can be held in place on the torso 114 using any suitable technique or techniques, e.g., adhesives, hydrogels, wrap-around tension bands connected to the arm, medical-grade tapes, etc.

Disposed on the arm 104 are one or more sensors 122. Each sensor 122 is adapted to sense bioelectric data when in contact with skin of the patient 116. The sensors 122 can be positioned or formed on at least one of the first major surface 132 or the second major surface 134 of the substrate 126. In one or more embodiments, one or more sensors 122 can be disposed on the second major surface 134 of the substrate 126 such that the sensors are in contact with the skin of the patient 116. In one or more embodiments, the sensors 122 can be positioned or formed on the first major surface 132 of the substrate 126, and one or more openings or vias can be formed in the substrate that coincide with the sensors such that the sensors can contact skin of the patient 116. In one or more embodiments, one or more of the sensors can be disposed within the substrate 126 as shown in FIG. 6. The sensors 122 can include any suitable sensor that is adapted to sense bioelectric data when in contact with skin of the patient 116. Any suitable number of sensors 122 can be disposed on the arm 104. Further, the sensors 122 can be disposed in any suitable arrangement on any suitable portion of the arm 104. In one or more embodiments, the sensors 122 are disposed on the arm 104 along the arm axis 106.

In one or more embodiments, the device 100 can be configured such that the sensors 122 surround the heart of the patient 116 and record, or monitor, the electrical signals associated with the depolarization and repolarization of the heart after the signals have propagated through the torso of the patient. Each of the sensors 122 can be used in a unipolar configuration to sense torso surface potentials that reflect the cardiac signals. In one or more embodiments, the sensors 122 can be used to evaluate electrical dyssynchrony in the heart of the patient. In such embodiments, the sensors 122 can be positioned over the torso 114 of the patient 116, including, e.g., the anterior 118, lateral 112, and posterior surfaces 120 of the torso 114 of the patient 116. A medical monitoring system or apparatus (e.g., apparatus 10 of FIG. 1) connected to the connector 124 of the device 100 can record and analyze the torso surface potential signals sensed by the sensors.

The sensors 122 can be formed using any suitable technique or techniques. For example, in one or more embodiments, the sensors 122 can be formed on the substrate 126 using flexographic printing with conductive inks such as Ag, AgCl, copper, Ag flakes in a flexible polymer, etc. In one or more embodiments, the sensors can be formed by chemically etching one or more metals.

The sensors 122 can be positioned in any suitable arrangement on the arm 104. As shown in FIG. 2, at least one of the sensors 122 is disposed on the arm 104 along the arm axis 106. Further, one or more of the sensors 122 can be removed from the arm 104 utilizing any suitable technique or techniques, e.g., the techniques described in co-owned U.S. Patent No. 9,320,446 B2, entitled BIOELECTRIC SENSOR DEVICE AND METHODS. Such removal of one or more sensors 122 can be utilized to accommodate unique anatomical features of each patient.

The sensors 122 can be electrically connected to the connector 124 using any suitable technique or techniques. In one or more embodiments, one or more conductors 136 can extend from each sensor 122 to the connector 124. The connector 124 can include one or more contacts, where each contact is electrically connected to a sensor 122. As shown in FIG. 6, the conductors 136 can be disposed on or within the substrate 126. In one or more embodiments, one or more conductors 136 can be disposed on the second major surface 134 of the substrate 126. Further, in one or more embodiments, one or more conductors 136 can be disposed on the first major surface 132. In one or more embodiments, one or more conductors 136 can be disposed on the first major surface 132 of the substrate 126 and one or more conductors can be disposed on the second major surface 134 of the substrate. In one or more embodiments, one or more vias can be formed through the substrate 126 to connect a conductor 136 disposed on the first major surface 132 of the substrate 126 or within the substrate to a sensor 122 disposed on the second major surface 134 of the substrate.

The conductors 136 can include any suitable conductive material or materials, e.g., at least one of metal, carbon, or graphite. In one or more embodiments, nanotubes or conductive flakes or particles (e.g., formed of at least one of metal (e.g., Ag, AgCl, copper, Ag flakes disposed in a flexible polymer), carbon, graphite, or other suitable conductive materials) can act as a conductor and be provided within a matrix or carrier. In one or more embodiments, the conductors 136 can include an insulating coating that may be provided on or over the conductive material, where the coating can be made from electrically conductive material that can be used as a shielding layer to minimize any interference from unwanted transient signals. And the conductors 136 can take any suitable shape or shapes and include any suitable dimensions.

The conductors 136 can be formed using any suitable technique or techniques. For example, in one or more embodiments, the conductors 136 can be formed on the substrate 126 using flexographic printing with conductive inks or chemical etching of metals. In one or more embodiments, one or more conductors 136 can be disposed on the first major surface 132 of the substrate 126 and one or more additional conductors can be disposed on the second major surface 134 of the substrate. The conductors 136 can be formed in any suitable pattern.

Electrically connected to the sensors 122 is the connector 124. The device 100 can include any suitable connector or connectors that are adapted to electrically connect the sensors 122 to a system such as system 10 of FIG. 1. The connector 124 can be disposed in any suitable relationship relative to the central portion 102 and the arm 104. In the embodiment illustrated in FIGS. 1-6, the connector 124 is connected to the central portion 102 of the device 100. As mentioned herein, the connector 124 can include one or more contacts, where each contact is electrically connected to a sensor 122.

As mentioned herein, the device 100 can include any suitable number of arms that extend from the central portion 102 or from another arm. According to the claimed invention, the device 100 includes a first arm 104, a second arm 138, a third arm 146 and fourth arm 150, the four arms extending from the central portion 102. For example, the device 100 includes a second arm 138 that extends from the central portion 102. All of the design considerations regarding the first arm 104 apply equally to the second arm 138. At least a portion 139 of the second arm 138 extends along a second arm axis 140. In one or more embodiments, the entire second arm 138 extends along the second arm axis 140. Further, in one or more embodiments, a second portion 141 of the second arm 138 does not extend along the second arm axis 140.

The second arm 138 includes one or more sensors 142 disposed on or in the second arm along the second arm axis 140. The sensors 142 can include any suitable sensor, e.g., the same sensors described herein regarding sensors 122.

The second arm 138 can be connected to the central portion 102 using any suitable technique or techniques. In one or more embodiments, the second arm 138 is integral with the central portion 102. Further, in one or more embodiments, the second arm 138 is manufactured separately and connected to the central portion 102 using any suitable technique or techniques, e.g., fastener 101.

The second arm 138 can extend from the central portion 102 in any suitable relationship to the arm 104. In one or more embodiments, the arm axis 106 of the arm 104 is substantially parallel to the second arm axis 140 of the second arm 138. As used herein, the term "substantially parallel" means that an angle formed between two arm axes is less than 10 degrees. In one or more embodiments, the arm axis 106 of the arm 104 and the second arm axis 140 of the second arm 138 are colinear.

The second arm 138 can be disposed on any suitable portion or portions of the torso 114 of the patient 116. In one or more embodiments, the arm 104 can be adapted to be disposed on the anterior surface 118 of the torso 114 and the second arm 138 can be adapted to be disposed on the posterior surface 120 of the torso.

The electrodes 142 disposed on or within this second arm 138 can be electrically connected to the connector 124 using any suitable technique or techniques. In one or more embodiments, each electrode 142 of the second arm 138 is electrically connected to the connector 124 by a conductor 144. Each electrode 142 of the second arm 138 can be electrically connected to a contact of the connector 124 by the conductor 144. In embodiments where the second arm is manufactured separately and then connected to the central portion 102, the second arm 138 can include a connector as is further described herein that electrically connects the electrodes 142 of the second arm to the connector 124 or two a patient monitoring apparatus or system.

In the embodiment illustrated in FIGS. 2-6, the device 100 includes at least a third arm 146 and a fourth arm 150. Each of the third and fourth arms 146, 150 extends from the central portion 102. At least a portion 147 of the third arm 146 extends along the third arm axis 148, and at least a portion 151 of the fourth arm 150 extends along the fourth arm axis 152. All of the design considerations and possibilities regarding the arm 104 apply equally to the third and fourth arms 146, 150.

As shown in FIG. 2, one or more sensors 154 are disposed on or within the third arm 146 along the third arm axis 148 and one or more sensors 156 are disposed on or within the fourth arm 150 along the fourth arm axis 152. Further, each of the third and fourth arms 146, 150 can include a connector electrically connected to the respective sensors 154, 156 as is further described herein. In one or more embodiments, conductors 158 can electrically connect each of the sensors 154 of the third arm 146 to a contact in the connector 124, and conductors 160 can electrically connect each of the sensors 156 of the fourth arm 150 to a contact in the connector.

The arms 104, 138, 146, and 150 of the device 100 (collectively arms 103) can extend from the central portion 102 in any suitable arrangement relative to each other. For example, in the embodiment illustrate in FIGS. 2-6, the arm axis 106 of the first arm 104 and the third arm axis 148 of the third arm 146 are substantially parallel. Further, the second arm axis 140 of the second arm 138 is substantially parallel to the fourth arm axis 152 of the fourth arm 150.

Each of the arms 103 can be adapted to be disposed on any portion of the torso 114 of the patient 116. In one or more embodiments, the arm 104 and the third arm 146 are adapted to be disposed on the anterior portion 118 of the torso 114, and the second arm 138 and the fourth arm 150 are adapted to be disposed on the posterior portion 120 of the torso.

The device 100 can further include one or more reference electrodes 162. Any suitable electrode can be utilized for the reference electrodes 162, e.g., the same electrodes described herein disposed on one or more of the arms 103. In one or more embodiments, each reference electrode 162 can be disposed on or within a substrate and connected to the torso 114 utilizing an adhesive (e.g., adhesive layer 170) such as a conductive adhesive. A liner such as liner 133 can be disposed on the adhesive such that the adhesive is disposed between the reference electrode 162 and the liner.

Such reference electrodes 162 can be disposed in any suitable relationship relative to the central portion 102 and arms 103 of the device 100. In the embodiment illustrated in FIGS. 2-6, the reference electrodes 162 are connected to the third and fourth arms 146, 150. The reference electrodes 162 can be integral with the second and fourth arms 146, 150, i.e., the substrate 126 can in part form the reference electrodes. In one or more embodiments, one or more of the reference electrodes 162 can be manufactured separately and connected to the second and fourth arms 146, 150 using any suitable technique or techniques. The reference electrodes 162 are adapted to be disposed on any suitable portion of the torso 114 of the patient 116.

Each of the reference electrodes 162 is electrically connected to the connector 124 by a conductor 164. In one or more embodiments, each reference electrode 162 can be electrically connected to a contact associated with the connector 124 by the conductor 164.

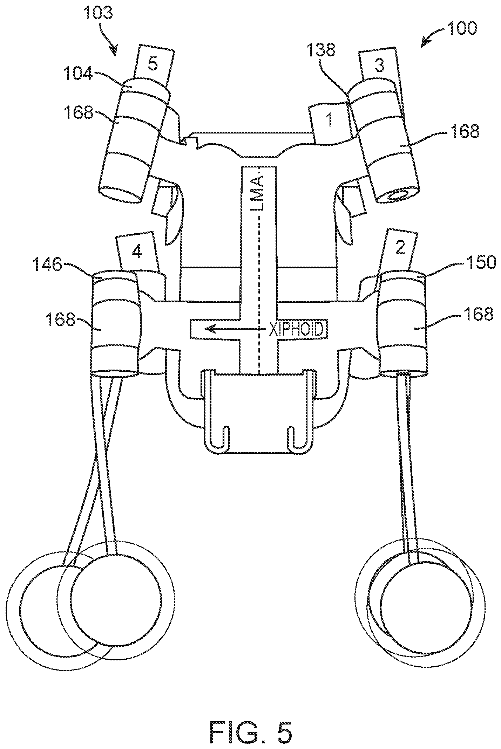

One or more embodiments of the device 100 can be adapted to be folded or rolled together for packaging. For example, FIG. 5 is a schematic plan view of the device 100 with each arm 103 in the undeployed configuration. As shown in FIG. 5, each of the arms 103 is adapted to be folded or rolled up. In one or more embodiments, one or more retainers 168 can be utilized to keep the arms 103 in a folded or rolled-up when in the undeployed configuration. When in the undeployed configuration, one or more of the arms 103 are folded. In one or more embodiments, one or more of the arms 103 are rolled up when in the undeployed configuration The bioelectric sensor device according to the claimed invention comprises a retainer for each of the first, second, third and fourth arm adapted to releasably fix the first, second, third and fourth arm in an undeployed configuration, wherein the first, second, third and fourth arms are folded or rolled up when in the undeployed configuration. The retainer 168 can include any suitable fastener, e.g., a strap having a fastening member such as a snap or hook-and-loop fastener, tape, repositionable adhesive tape, buttons, paper clips, rivets, rings, cord locks, hook-and-eye fasteners, magnets, ties, zippers, frog fasteners, etc. The retainer 168 can be removed from its respective arm 103 such that the arm is in the deployed configuration as shown in FIG. 3.

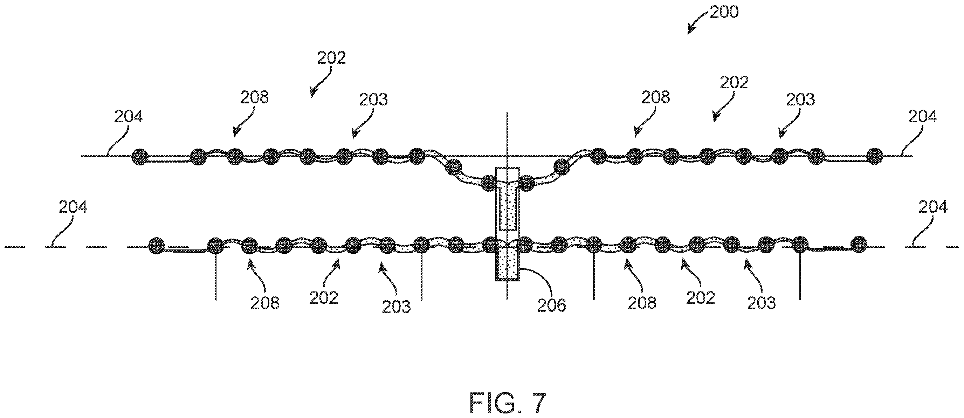

As mentioned herein, the one or more arms of the various embodiments of bioelectric sensor devices can take any suitable shape or shapes. For example, FIG. 7 is a schematic plan view of another embodiment of a bioelectric sensor device 200. All of the design considerations and possibilities regarding the bioelectric sensor device 100 of FIGS. 2-6 apply equally to the bioelectric sensor device 200 of FIG. 7.

One difference between the device 200 of FIG. 7 and device 100 of FIGS. 2-6 is that at least a portion 203 of one or more of arms 202 extends from a central portion 206 along an arm axis 204 and takes a serpentine shape that oscillates about the arm axis. Such serpentine shape can provide extensibility and flexibility to the arms 202 such that an arm or arms can be repositioned along the arm axis 204 to accommodate and conform to different sizes of patients. Further, each arm 202 includes one or more sensors 208 that are disposed on the arm along the arm axis 204. As a result, the sensors 208 can remain aligned along the arm axis 204 while the arm 202 can take the described serpentine shape. Any suitable serpentine shape can be utilized, e.g., a zig-zag (i.e., triangular-wave) shape, a sinusoidal shape, etc. In one or more embodiments, one or more of the arms 202 can be formed to include one or more accordion folds such that the arm can be extended to accommodate various patient anatomies.

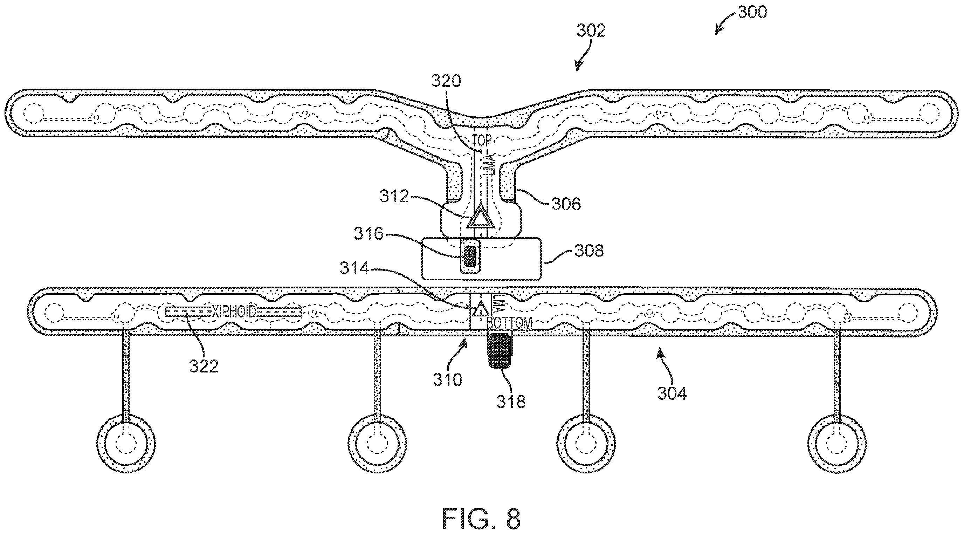

As is also mentioned herein, one or more arms of the various embodiments of bioelectric sensor devices can be integral with a central portion of the device or can be manufactured separately and then connected to the central portion using any suitable technique or techniques. For example, FIG. 8 is a schematic plan view of another embodiment of a bioelectric sensor device 300. All of the design considerations and possibilities regarding the bioelectric sensor device 100 of FIGS. 2-6 and the bioelectric sensor device 200 of FIG. 7 apply equally to the device 300 of FIG. 8.

One difference between device 300 and device 100 is that device 300 includes a first segment 302 and a second segment 304. In one or more embodiments, the first and second segments 302, 304 can be connected together using any suitable technique or techniques, e.g., adhering, mechanically fastening, etc. In one or more embodiments, a central portion 306 of the first segment 302 can include a first attachment region 308, and the second segment 304 can include a second attachment region 310 that is adapted to be connected to the first attachment region. The first and second attachment regions 308, 310 can include any suitable attachment mechanisms to connect the first and second portions 302, 304, e.g., the same fasteners described herein regarding 101 of FIG. 2. In one or more embodiments, at least one of the first attachment region 308 and the second attachment region 310 can include an adhesive layer such as a repositionable adhesive layer that can connect the first and second segments 302, 304.

Another difference between device 300 and device 100 is that the first segment 302 further includes a first attachment mark 312 and the second segment 304 includes a second attachment mark 314. The first and second attachment marks 312, 314 are adapted to assist a user in aligning the first and second segments 302, 304 when being disposed on a torso of a patient and connecting the segments together. Further, the first and second attachment marks 312, 314 can be utilized to provide spatial and rotational alignment of the first and second segments 302, 304 when being disposed on the torso. The first and second attachment marks 312, 314 can include any suitable markings or indicia, e.g., the same markings described herein regarding anatomical alignment mark 108 of device 100.

Further, unlike the device 100, the first segment 302 of device 300 includes a first connector 316 and the second segment 304 includes a second connector 318. The first and second connectors 316, 318 can include any suitable connector or connectors. In one or more embodiments, the first connector 316 is adapted to be connected to the second connector 318. In one or more embodiments, the first connector 316 is adapted to be connected to a cable or connector of a patient monitoring system, and the second connector 318 is adapted to be separately connected to the cable or connector of the system.

The device 300 can further include one or more anatomical alignment marks. As shown in FIG. 8, the device 300 includes a first anatomical alignment mark 320 and a second anatomical alignment mark 322. The first and second anatomical alignment marks 320, 322 can include any suitable alignment marks, e.g., the same alignment marks described herein regarding anatomical alignment mark 108 of device 100. Although depicted as including two alignment marks 320, 322, the device 300 can include any suitable number of alignment marks.

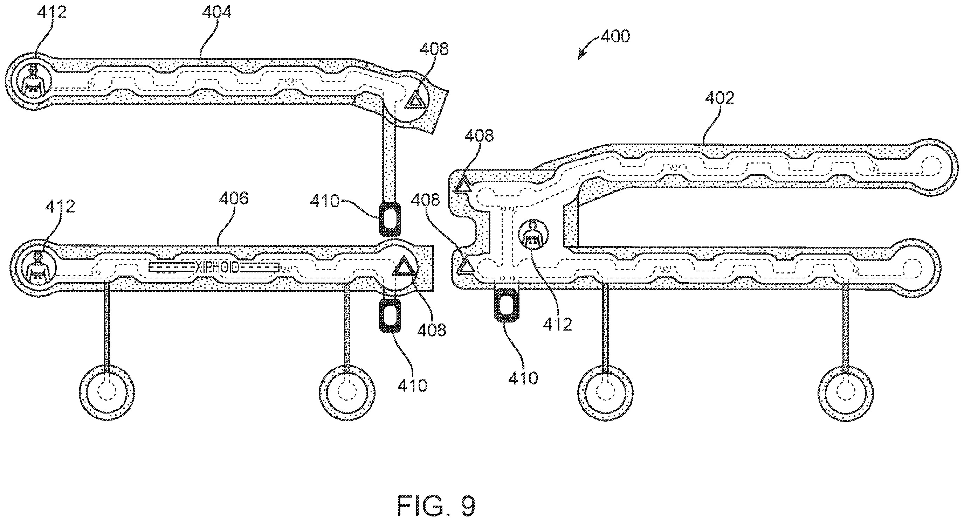

The device 300 can include any suitable number of segments that are connected utilizing any suitable technique or techniques. For example, FIG. 9 is a schematic plan view of another embodiment of a bioelectric sensor device 400. All of the design considerations and possibilities regarding the device 100 of FIGS. 2-6, the device 200 of FIG. 7, and the device 300 of FIG. 8 apply equally to the device 400 of FIG. 9.

As shown in FIG. 9, the device 400 includes a first segment 402, a second segment 404, and a third segment 406. The segments 402, 404, 406 can be connected utilizing any suitable technique or techniques, e.g., the same techniques described herein regarding device 300 of FIG. 8. Further, the device 400 can include one or more alignment marks 408 that can assist a user in connecting the second and third segments 404, 406 to the first segment 402. In one or more embodiments, the alignment marks 408 utilized to connect the second segment 404 to the first segment 402 can be different from the alignment marks utilized to connect the third segment 406 to the first segment 402.

One difference between the device 400 of FIG. 9 and the device 100 of FIGS. 2-6 is that the device 400 includes multiple connectors 410. In one or more embodiments, the connectors 410 can be connected together to provide a single connector that is adapted to be connected to a cable of a patient monitoring system or directly to the patient monitoring system utilizing any suitable technique or techniques. In one or more embodiments, each connector 410 can be adapted to connect directly to the cable of the patient monitoring system or to the system without first being connected.

The device 400 further includes one or more images 412 disposed on one or more of the segments 402, 404, 406. Such images 412 can include any suitable images or markings that can assist the user in disposing the segments 402, 404, 406 on a torso of a patient. For example, in one or more embodiments, one or more images 412 can graphically indicate to the user where a segment of the device 400 can be placed on the torso.

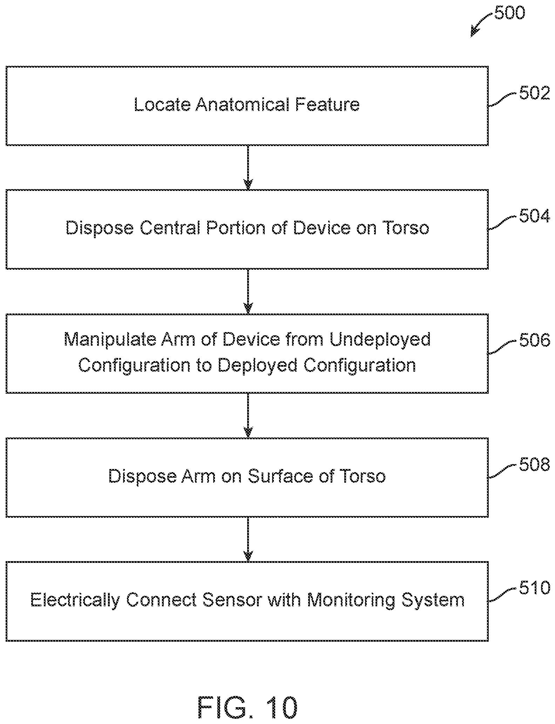

Any suitable technique or techniques can be utilized to dispose the various embodiments of bioelectric sensor devices described herein on a torso of a patient. For example, FIG. 10 is a flowchart of one embodiment of a method 500 of disposing the device 100 on the torso 114 of the patient 116. Although described in regard to the device 100, the method 500 can be utilized with any suitable bioelectric sensor device. At 502, the anatomical feature 110 on the torso 114 can be located utilizing any suitable technique or techniques. In one or more embodiments, the anatomical feature 110 can be disposed on the lateral surface 112 of the torso 114. The central portion 102 of the device 100 can be disposed on the lateral surface 112 of the torso 114 at 504 utilizing any suitable technique or techniques such that the anatomical alignment mark 108 disposed on the central portion is aligned with the anatomical feature. In one or more embodiments, the central portion 102 can be disposed by removing the liner 133 from the adhesive layer 170 that is disposed on the second major surface 134 of the substrate 126 of the central portion, and attaching the central portion to the lateral surface 112 of the torso 114.

At 506, one or more of the arms 103 of the device 100 can be manipulated from the undeployed configuration (FIG. 5) to the deployed configuration (FIG. 4) using any suitable technique or techniques. In one or more embodiments, one or more of the arms 103 can be manipulated by removing the retainer 168 from the arm and unrolling the arm such that it is in the deployed configuration.

At 508, one or more of the arms 103 can be disposed on either the anterior surface 118 or posterior surface 120 of the torso 114 of the patient 116 utilizing any suitable technique or techniques. In one or more embodiments, each of the arms 103 can be deployed by removing the liner 133 from the conductive adhesive layer 170 and attaching the arm to either the anterior surface 118 or the posterior surface 120 of the torso 114.

At 510, one or more of the sensors 122, 138, 154, 156 can be electrically connected to a patient monitoring system (e.g., monitoring system 10) utilizing any suitable technique or techniques. For example, the connector 124 can be connected to a cable of the monitoring system or directly to the monitoring system.

In one or more embodiments, one or more of the reference electrodes 162 can be disposed on a surface of the torso 114 utilizing any suitable technique or techniques. For example, a liner can be removed from a conductive adhesive layer disposed on a substrate of the reference electrode 162. The reference electrode 162 can be connected to the surface of the torso 114 with the conductive adhesive.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include computer-readable storage media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A bioelectric sensor device (100) comprising:
a central portion (102) having a left edge (128) and a right edge (130);
a first arm (104) extending from the left edge (128) of the central portion, wherein at least a portion (105) of the first arm extends along a first arm axis (106), wherein the central portion comprises an anatomical alignment mark (108) adapted to align the central portion with an anatomical feature (110) of a lateral surface (112) of a torso (114) of a patient (116), and further wherein the first arm is adapted to be disposed on an anterior (118) or posterior (120) surface of the torso;
a sensor (122) disposed on the first arm along the first arm axis; and
a connector (124) electrically connected to the sensor
a second arm (138) extending from the right edge (130) of the central portion, wherein at least a portion (139) of the second arm extends along a second arm axis (140); and
a sensor (142) disposed on the second arm along the second arm axis and electrically connected to the connector
a third arm (146) extending from the left edge (128) of the central portion, wherein at least a portion (147) of the third arm extends along a third arm axis (148);
a sensor (154) disposed on the third arm along the third arm axis and electrically connected to the connector
a fourth arm (150) extending from the right edge (130) of the central portion, wherein at least a portion (151) of the fourth arm extends along a fourth arm axis (152);
a sensor (156) disposed on the fourth arm along the fourth arm axis and electrically connected to the connector;
**characterized in that** the sensor device further comprises:
a retainer for each of the first, second, third and fourth arm adapted to releasably fix the first, second, third and fourth arm in an undeployed configuration, wherein the first, second, third and fourth arms are folded or rolled up when in the undeployed configuration.

2. The device of claim 1, wherein the arm comprises a serpentine shape that oscillates about the arm axis.

3. The device of any one of claims 1-2, wherein the first arm is integral with the central portion and/or wherein the second arm and fourth arm are integral with the central portion.

4. The device of any one of claims 1-2, wherein the first arm is connected to the central portion by a fastener (101), wherein a first alignment mark disposed on the first arm and a second alignment mark disposed on the central portion are adapted to be aligned when the first arm is connected to the central portion.

5. The device of any of the claims 1-4, wherein the first arm axis and the second arm axis are substantially parallel and/or wherein the first arm axis and the second arm axis are substantially colinear.

6. The device of any one of claims 1-5, wherein the first arm is adapted to be disposed on the anterior surface of the torso and the second arm is adapted to be disposed on the posterior surface of the torso.

7. The device of any of the claims 1-6, wherein the first arm axis and the third arm axis are substantially parallel.

8. The device of any one of claims 1-7, wherein the second arm axis and the fourth arm axis are substantially parallel.

9. The device of any one of claims 1-8, wherein the first arm and third arm are adapted to be disposed on the anterior surface of the torso, and the second arm and fourth arm are adapted to be disposed on the posterior surface of the torso.

10. The device of any one of claims 1-9, further comprising a reference electrode connected (162) to the first arm, wherein the reference electrode is electrically connected to the connector.

11. The device of any one of claims 1-10, further comprising a second anatomical alignment mark (322) disposed on the first arm.

12. The device of any one of claims 1-11, wherein the retainer comprises a strap comprising a fastening member.

13. The device of any one of claims 1-12, wherein the arm further comprises a substrate (126) and a conductor (136) disposed on the substrate, wherein the conductor electrically connects the sensor to the connector, particularly wherein the arm further comprises a conductive adhesive layer (170) disposed on a major surface (134) of the substrate and a liner (133) disposed on the conductive adhesive layer such that the adhesive layer is disposed between the substrate and the liner.

14. The device of any one of claims 1-13, wherein the arm further comprises a plurality of sensors each electrically connected to the connector.

15. A patient management system (10) comprising the bioelectric sensor device of any one of claims 1-14.

## Patentansprüche

1. Bioelektrische Sensorvorrichtung (100), umfassend:
einen Mittelabschnitt (102) mit einer linken Kante (128) und einer rechten Kante (130);
einen ersten Arm (104), der sich von der linken Kante (128) des Mittelteils erstreckt, wobei sich mindestens ein Abschnitt (105) des ersten Arms entlang einer ersten Armachse (106) erstreckt, wobei der Mittelabschnitt eine anatomische Ausrichtungsmarkierung (108) umfasst, die dazu geeignet ist, den Mittelabschnitt mit einem anatomischen Merkmal (110) einer seitlichen Oberfläche (112) eines Rumpfes (114) eines Patienten (116) auszurichten, und wobei ferner der erste Arm dazu geeignet ist, auf einer anterioren (118) oder posterioren (120) Oberfläche des Rumpfes angeordnet zu werden;
einen Sensor (122), der am ersten Arm entlang der ersten Armachse angeordnet ist; und
einen Verbinder (124), der elektrisch mit dem Sensor verbunden ist,
einen zweiten Arm (138), der sich von der rechten Kante (130) des Mittelabschnitts erstreckt, wobei sich zumindest ein Abschnitt (139) des zweiten Arms entlang einer zweiten Armachse (140) erstreckt; und
einen Sensor (142), der am zweiten Arm entlang der zweiten Armachse angeordnet und elektrisch mit dem Verbinder verbunden ist,
einen dritten Arm (146), der sich von der linken Kante (128) des Mittelabschnitts erstreckt, wobei sich mindestens ein Abschnitt (147) des dritten Arms entlang einer dritten Armachse (148) erstreckt;
einen Sensor (154), der am dritten Arm entlang der dritten Armachse angeordnet und elektrisch mit dem Verbinder verbunden ist,
einen vierten Arm (150), der sich von der rechten Kante (130) des Mittelabschnitts erstreckt, wobei sich mindestens ein Abschnitt (151) des vierten Arms entlang einer vierten Armachse (152) erstreckt;
einen Sensor (156), der auf dem vierten Arm entlang der vierten Armachse angeordnet und elektrisch mit dem Verbinder verbunden ist;
**dadurch gekennzeichnet, dass** die Sensorvorrichtung ferner umfasst:
eine Halterung für jeden der ersten, zweiten, dritten und vierten Arme, die dazu geeignet ist, den ersten, zweiten, dritten und vierten Arm in einer nicht ausgefahrenen Konfiguration lösbar zu fixieren, wobei der erste, zweite, dritte und vierte Arm in der nicht ausgefahrenen Konfiguration gefaltet oder aufgerollt sind.

2. Vorrichtung nach Anspruch 1, wobei der Arm eine Serpentinenform aufweist, die um die Armachse schwingt.

3. Vorrichtung nach einem der Ansprüche 1 bis 2, wobei der erste Arm einstückig mit dem Mittelabschnitt ausgebildet ist und/oder wobei der zweite und der vierte Arm einstückig mit dem Mittelabschnitt ausgebildet sind.

4. Vorrichtung nach einem der Ansprüche 1 bis 2, wobei der erste Arm durch ein Befestigungselement (101) mit dem Mittelabschnitt verbunden ist, wobei eine auf dem ersten Arm angeordnete erste Ausrichtungsmarkierung und eine auf dem Mittelabschnitt angeordnete zweite Ausrichtungsmarkierung so angepasst sind, dass sie ausgerichtet werden, wenn der erste Arm mit dem Mittelabschnitt verbunden ist.

5. Vorrichtung nach einem der Ansprüche 1 bis 4, wobei die erste Armachse und die zweite Armachse im Wesentlichen parallel sind und/oder wobei die erste Armachse und die zweite Armachse im Wesentlichen kolinear sind.

6. Vorrichtung nach einem der Ansprüche 1 bis 5, wobei der erste Arm zur Anordnung an der anterioren Oberfläche des Rumpfes und der zweite Arm zur Anordnung an der posterioren Oberfläche des Rumpfes geeignet ist.

7. Vorrichtung nach einem der Ansprüche 1 bis 6, wobei die erste Armachse und die dritte Armachse im Wesentlichen parallel sind.

8. Vorrichtung nach einem der Ansprüche 1 bis 7, wobei die zweite Armachse und die vierte Armachse im Wesentlichen parallel sind.

9. Vorrichtung nach einem der Ansprüche 1 bis 8, wobei der erste Arm und der dritte Arm dazu geeignet sind, auf der anterioren Oberfläche des Rumpfes angeordnet zu werden, und der zweite Arm und der vierte Arm dazu geeignet sind, auf der posterioren Oberfläche des Rumpfes angeordnet zu werden.

10. Vorrichtung nach einem der Ansprüche 1 bis 9, ferner umfassend eine Bezugselektrode, die mit dem ersten Arm verbunden (162) ist, wobei die Bezugselektrode elektrisch mit dem Verbinder verbunden ist.

11. Vorrichtung nach einem der Ansprüche 1 bis 10, ferner umfassend eine zweite anatomische Ausrichtungsmarkierung (322), die am ersten Arm angeordnet ist.

12. Vorrichtung nach einem der Ansprüche 1 bis 11, wobei die Halterung einen Riemen mit einem Befestigungselement umfasst.

13. Vorrichtung nach einem der Ansprüche 1 bis 12, wobei der Arm ferner ein Substrat (126) und einen auf dem Substrat angeordneten Leiter (136) umfasst, wobei der Leiter den Sensor elektrisch mit dem Verbinder verbindet, insbesondere wobei der Arm ferner eine auf einer Hauptoberfläche (134) des Substrats angeordnete leitfähige Klebeschicht (170) und eine auf der leitfähigen Klebeschicht angeordnete Auskleidung (133) umfasst, so dass die Klebeschicht zwischen dem Substrat und der Auskleidung angeordnet ist.

14. Vorrichtung nach einem der Ansprüche 1 bis 13, wobei der Arm ferner eine Vielzahl von Sensoren umfasst, die jeweils elektrisch mit dem Verbinder verbunden sind.

15. Patientenverwaltungssystem (10), das die bioelektrische Sensorvorrichtung nach einem der Ansprüche 1 bis 14 umfasst.

## Revendications

1. Dispositif de capteur bioélectrique (100) comprenant :
une partie centrale (102) ayant un bord gauche (128) et un bord droit (130) ;
un premier bras (104) s'étendant à partir du bord gauche (128) de la partie centrale, dans lequel au moins une partie (105) du premier bras s'étend le long d'un axe du premier bras (106), dans lequel la partie centrale comprend une marque d'alignement anatomique (108) adaptée pour aligner la partie centrale avec une caractéristique anatomique (110) d'une surface latérale (112) d'un torse (114) d'un patient (116), et en outre dans lequel le premier bras est adapté pour être disposé sur une surface antérieure (118) ou postérieure (120) du torse ;
un capteur (122) disposé sur le premier bras le long de l'axe du premier bras ; et
un connecteur (124) connecté électriquement au capteur
un deuxième bras (138) s'étendant à partir du bord droit (130) de la partie centrale, dans lequel au moins une partie (139) du deuxième bras s'étend le long d'un axe du deuxième bras (140) ; et
un capteur (142) disposé sur le deuxième bras le long de l'axe du deuxième bras et connecté électriquement au connecteur
un troisième bras (146) s'étendant à partir du bord gauche (128) de la partie centrale, dans lequel au moins une partie (147) du troisième bras s'étend le long d'un axe du troisième bras (148) ;
un capteur (154) disposé sur le troisième bras le long de l'axe du troisième bras et connecté électriquement au connecteur
un quatrième bras (150) s'étendant à partir du bord droit (130) de la partie centrale, dans lequel au moins une partie (151) du quatrième bras s'étend le long d'un axe du quatrième bras (152) ;
un capteur (156) disposé sur le quatrième bras le long de l'axe du quatrième bras et connecté électriquement au connecteur ;
**caractérisé en ce que** le dispositif de capteur comprend en outre :
un dispositif de retenue pour chacun des premier, deuxième, troisième et quatrième bras, adapté pour fixer de manière amovible les premier, deuxième, troisième et quatrième bras dans une configuration non déployée, dans laquelle les premier, deuxième, troisième et quatrième bras sont pliés ou enroulés lorsqu'ils sont dans la configuration non déployée.

2. Dispositif selon la revendication 1, dans lequel le bras comprend une forme serpentine qui oscille autour de l'axe du bras.

3. Dispositif selon l'une quelconque des revendications 1 à 2, dans lequel le premier bras fait partie intégrante de la partie centrale et/ou dans lequel le deuxième bras et le quatrième bras font partie intégrante de la partie centrale.

4. Dispositif selon l'une quelconque des revendications 1 à 2, dans lequel le premier bras est relié à la partie centrale par une attache (101), dans lequel une première marque d'alignement disposée sur le premier bras et une seconde marque d'alignement disposée sur la partie centrale sont adaptées pour être alignées lorsque le premier bras est relié à la partie centrale.

5. Dispositif selon l'une quelconque des revendications 1 à 4, dans lequel l'axe du premier bras et l'axe du deuxième bras sont sensiblement parallèles et/ou dans lequel l'axe du premier bras et l'axe du deuxième bras sont sensiblement colinéaires.

6. Dispositif selon l'une quelconque des revendications 1 à 5, dans lequel le premier bras est adapté pour être disposé sur la surface antérieure du torse et le second bras est adapté pour être disposé sur la surface postérieure du torse.

7. Dispositif selon l'une quelconque des revendications 1 à 6, dans lequel l'axe du premier bras et l'axe du troisième bras sont sensiblement parallèles.

8. Dispositif selon l'une quelconque des revendications 1 à 7, dans lequel l'axe du deuxième bras et l'axe du quatrième bras sont sensiblement parallèles.

9. Dispositif selon l'une quelconque des revendications 1 à 8, dans lequel le premier bras et le troisième bras sont adaptés pour être disposés sur la surface antérieure du torse, et le deuxième bras et le quatrième bras sont adaptés pour être disposés sur la surface postérieure du torse.

10. Dispositif selon l'une quelconque revendications 1 à 9, comprenant en outre une électrode de référence connectée (162) au premier bras, dans lequel l'électrode de référence est connectée électriquement au connecteur.

11. Dispositif selon l'une quelconque des revendications 1 à 10, comprenant en outre une seconde marque d'alignement anatomique (322) disposée sur le premier bras.

12. Dispositif selon l'une quelconque des revendications 1 à 11, dans lequel le dispositif de retenue comprend une sangle comprenant un élément de fixation.

13. Dispositif selon l'une quelconque des revendications 1 à 12, dans lequel le bras comprend en outre un substrat (126) et un conducteur (136) disposé sur le substrat, dans lequel le conducteur connecte électriquement le capteur au connecteur, en particulier dans lequel le bras comprend en outre une couche adhésive conductrice (170) disposée sur une surface principale (134) du substrat et un revêtement (133) disposé sur la couche adhésive conductrice de telle sorte que la couche adhésive est disposée entre le substrat et le revêtement.

14. Dispositif selon l'une quelconque des revendications 1 à 13, dans lequel le bras comprend en outre une pluralité de capteurs, chacun connecté électriquement au connecteur.

15. Système de gestion de patients (10) comprenant le dispositif de capteur bioélectrique selon l'une quelconque des revendications 1 à 14.
